# EUROPÄISCHE PATENTSCHRIFT

(11) **EP 3 451 943 B1**
(45) Veröffentlichungstag und Bekanntmachung des Hinweises auf die Patenterteilung: **04.12.2019**
(21) Anmeldenummer: 18731050.3
(22) Anmeldetag: 11.06.2018
(51) Int. Cl.: A61B 17/14

(54) **SÄGEBLATT**
SAW BLADE
LAME DE SCIE

(30) Priorität: 12.06.2017 DE 102017112872
(43) Veröffentlichungstag der Anmeldung: 13.03.2019
(73) Patentinhaber: Aesculap AG, 78532 Tuttlingen (DE)
(72) Erfinder: SCHAZ, Uwe, 78579 Neuhausen (DE); MATTES, Uwe, 78532 Tuttlingen (DE); SCHÄFER, Markus, 78073 Bad Dürrheim (DE); MOSER, Elke, 78588 Denkingen (DE)
(74) Vertreter: Winter, Brandl, Fürniss, Hübner, Röss, Kaiser, Polte - Partnerschaft mbB
(86) Internationale Anmeldenummer: PCT/EP2018/065326
(87) Internationale Veröffentlichungsnummer: WO 2018/228979

(56) Entgegenhaltungen:
- EP-A1- 2 974 677
- US-A1- 2004 138 670
- US-A1- 2006 009 796
- US-A1- 2006 123 959

## Beschreibung

Die vorliegende Erfindung betrifft ein auswechselbares chirurgisches Sägeblatt für eine oszillierende medizinische/chirurgische Sägemaschine gemäß dem Oberbegriff des Patentanspruchs 1.

### Hintergrund der Erfindung

In der modernen Chirurgie werden zum Durchtrennen von Knochen wie z.B. das Hüftgelenk, das Sternum, etc. elektrische Knochensägemaschinen verwendet, wie sie u.a. auch von der Anmelderin der vorliegenden Erfindung entwickelt und hergestellt werden. Eine solche Knochensägemaschine besteht aus einem Gehäuse, das gleichzeitig auch zum Haltegriff geformt ist und in dem eine Antriebseinrichtung zum oszillierenden Antrieb eines Schwingkopfes untergebracht ist. Der Schwingkopf hat eine Werkzeugaufnahme, in die ein Sägeblatt lösbar und damit auswechselbar eingesteckt ist.

Das Sägeblatt hat etwa die Form einer Zunge mit einer Sägezahnreihe an dessen zum Schwingkopf distalen Stirnkante, derart, dass bei einer oszillierenden Drehbewegung des Schwingkopfes die Sägezahnreihe oszillierend auf einer Kreisbahn bewegt wird und so eine Sägewirkung beispielsweise auf einen Knochen aufbringt.

### Stand der Technik

Aus dem Stand der Technik ist ein Sägeblatt der vorstehend definierten Gattung bekannt, wie es u.a. von der Anmelderin der vorliegenden Erfindung vertrieben wird. Dieses Sägeblatt hat in der Draufsicht eine im Wesentlichen rechteckige Form mit einem vorzugsweise als Ausstanzung ausgebildeten Kupplungsbereich an einem in Längsrichtung der Rechteckform liegenden Endabschnitt und einer Sägezahnreihe an der in Längsrichtung der Rechteckform gesehen anderen Stirnkante des Sägeblatts. Durch die bevorzugte Ausstanzung ergibt sich eine Art federnd verschwenkbare Rastzunge, die mit einem entsprechend gestaltenden Rast-/Clickmechanismus in lösbaren Rasteingriff bringbar ist, der an einer Werkzeugaufnahme einer allgemein bekannten Knochensägemaschine ausgebildet ist.

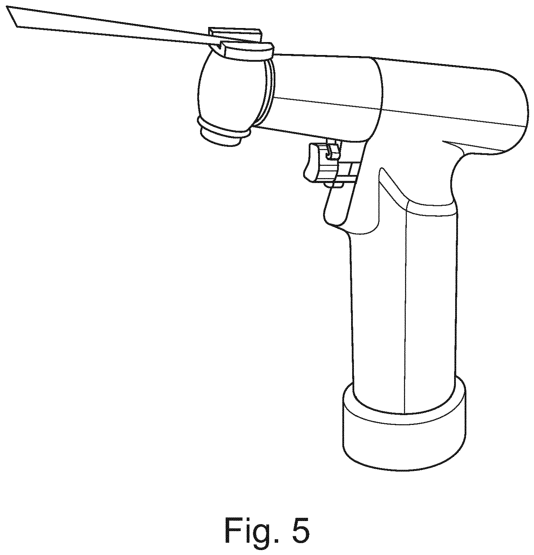

Zum besseren Verständnis ist in der Fig. 5 eine solche Knochensägemaschine dargestellt.

Demzufolge hat die bekannte Knochensägemaschine ein hülsenförmiges oder pistolenartiges Gehäuse, vorzugsweise aus einem Kunststoff, Aluminium-Material, Titan-Material oder einem Edelstahl, in dem ein nicht weiter dargestellter Antrieb, etwa ein Elektromotor samt zugehöriger Steuerungselektronik untergebracht ist. Am distalen Ende des Gehäuses ist ein Schwingkopf angeordnet an/in dem die Werkzeugaufnahme bzw. eine Einschubkupplung rotierbar gelagert ist. Über einen Getriebemechanismus wird die Antriebsbewegung des elektrischen Antriebs/Motors auf die Werkzeugaufnahme bzw. Einschubkupplung derart übertragen, dass diese eine oszillierende Rotationsbewegung mit einer vorbestimmten Frequenz ausführt.

Die Werkzeugaufnahme bildet vorzugsweise eine Art Schwalbenschwanzführung aus mit dem integrierten Click-/Rastmechanismus zwischen den beiden sich gegenüberliegenden Klemmschienen der Schwalbenschwanzführung. Wird somit das vorstehend beschriebene Sägeblatt in die Schwalbenschwanzführung in dessen Längsrichtung (zwischen den Klemmschienen der Schwalbenschwanzführung) eingeführt oder eingeschoben, kommt die Rastzunge schließlich mit dem Click-/Rastmechanismus in Eingriff und arretiert somit das Sägeblatt an der Werkzeugaufnahme.

Bei Inbetriebnahme der Knochensägemaschine wird die Werkzeugaufnahme in eine oszillierende Rotationsbewegung versetzt, die auf das Sägeblatt übertragen wird. Dadurch wird die Sägezahnreihe in eine Hin- und Herbewegung auf einer Kreisbahn versetzt, deren Radius dem Abstand zwischen der Drehachse der Werkzeugaufnahme und der distalen, freien Stirnkante des Sägeblatts entspricht.

Um die Handhabung der so aufgebauten Knochensägemaschine zu erleichtern, ist die distale, freie Stirnkante des Sägeblatts nicht geradlinig sondern bogenförmig entsprechend der vorstehend definierten Kreisbahn ausgebildet. Dadurch kann verhindert werden, dass sich die jeweils letzten Zähne der Sägezahnreihe im Knochenmaterial verhaken, was zu einer nicht zu kontrollierenden Rüttelbewegung der Knochensägemaschine führen könnte.

Es hat sich aber gezeigt, dass die vorstehende Formgebung des Sägeblatts in so weit nachteilig ist, als dass dieses beispielsweise beim Sägen eines Sternums nur schwierig unter der Patientenhaut längsgeführt werden kann. Selbst in dem Fall, dass das gerade Sägeblatt in einem Winkel zum Maschinengehäuse, beispielsweise ca. 45°, durch entsprechendes Verdrehen der Werkzeugaufnahme in Konstruktionslage ausgerichtet wird, kann das Sägeblatt nicht zufriedenstellend unter der Patientenhaut in Sägerichtung vorwärts getrieben werden, ohne die Patientenhaut zu verletzen. Hinzu kommt, dass die Knochensägemaschine entsprechende Einstellungen ermöglichen muss, was zu einer Verteuerung der Maschine führen würde.

Schließlich wurde festgestellt, dass unabhängig von der Winkelstellung des Sägeblatts bezüglich der Patientenhaut immer noch Weichteilverletzung auftreten könnten, insbesondere dann, wenn der letzte Sägezahn der Sägezahnreihe bei einer unkontrollierten Schwenkbewegung der Maschine in Kontakt mit Weichteilgewebe kommt und dieses dann zerschneidet.

Aus US 2006/0123959 ist ein blattförmiges Sägeblatt bekannt, bei dem die Aufnahmeeinrichtung zur Befestigung des Sägeblattes an einem Maschinengehäuse zu der Mittelachse, die senkrecht zur Sägezahnreihe am distalen Ende des Sägeblattes ist, schräg angeordnet ist.

US 2006/0009796 A1 offenbart ein Sägeblatt, welches eine L-Form aufweist, sodass die Sägezahnreihe des Sägeblattes und ein Kopplungsbereich in einem 90°-Winkel zueinander stehen.

Aus US 2004/0138670 ist ein einfach gewinkeltes Sägeblatt bekannt.

### Kurzbeschreibung der Erfindung

In Anbetracht der vorstehenden Problematik ist es die Aufgabe der vorliegenden Erfindung, ein Sägeblatt für eine (bzw. einer) Knochensägemaschine bereit zu stellen, die eine verbesserte Handhabbarkeit beispielsweise beim Sägen eines Sternums unter der Patientenhaut ermöglicht. Ein bevorzugtes Ziel ist es, das Risiko einer unbeabsichtigten Verletzung von Weichteilgewebe des Patienten weit möglichst zu minimieren.

Diese Aufgabe wird durch ein gattungsgemäßes Sägeblatt mit den Merkmalen das Patentanspruchs 1 gelöst. Vorteilhafte Ausgestaltungen der Erfindung sind Gegenstand der Unteransprüche.

Prinzipiell hat ein Sägeblatt für eine bzw. einer Knochensägemaschine einen proximal angeordneten oder ausgebildeten sowie eine Kopplungs- oder Einschubrichtung definierenden Kopplungsbereich, der zum Koppeln oder Einschieben des Sägeblatts mit/in eine(r) oszillierend um eine Rotationsachse rotierenden Werkzeugaufnahme der Knochensägemaschine vorgesehen und ausgebildet ist. Des Weiteren hat das Sägeblatt eine Sägezahnreihe, die an einer distalen Stirnkante des Sägeblatts angeordnet oder ausgebildet ist, derart, dass deren senkrecht zur Sägezahnreihe ausgerichtete Mittelachse den Kopplungsbereich des Sägeblatts und vorzugsweise die Rotationsachse im Wesentlichen schneidet, wenn das Sägeblatt mit der Werkzeugaufnahme (12) gekoppelt oder in diese eingeschoben ist. Dadurch bewegt sich die Sägezahnreihe im Wesentlichen tangential entlang einer Kreisbahn, wenn die Werkzeugaufnahme oszillierend rotiert wird. Ein Kern der vorliegenden Erfindung besteht nunmehr darin, dass die Mittelachse (M) und die Kupplungs- oder Einschubrichtung gemäß vorstehender Definitionen in einem Winkel ungleich 0° bzw. 180°, vorzugsweise in etwa 45° zueinander stehen bzw. ausgerichtet oder ausrichtbar sind.

Auf diese Weise ist es möglich, das Sägeblatt auch in solche Werkzeugaufnahmen einzuführen, deren definierte Einschubrichtung von der vorstehend definierten Mittelachse abweicht.

Dabei ist das Sägeblatt nicht gerade sondern mit zwei (gegenläufigen) Abkrümmungen etwa nach Art einer S-Form ausgebildet. Auf diese Weise ist es in besonders einfacher Weise möglich, die Sägerichtung des Sägeblatts über den einen freien Schenkel dieser im Wesentlichen S-Form (an welchem die Sägezahnreihe ausgeformt/ausgebildet ist) wählbar schräg bezüglich der Einschubrichtung der Werkzeugaufnahme (Einschubkupplung) anzustellen und gleichzeitig die von einer Knochensägemaschine vorgegebenen Winkelstellungen/Einschubrichtungen der Werkzeugaufnahme (Einschubkupplung) zu nutzen (ohne diese aufwändig verändern zu müssen), indem der andere freie Schenkel (an welchem der Kupplungsbereich, vorzugsweise die Rastzunge ausgebildet/angeordnet ist) entsprechend angestellt wird.

Ein die beiden freien Schenkel der quasi-S-Form verbindender Zwischensteg kann wählbar in einfacher Weise so dimensioniert sein/werden, dass eine geradlinige Verlängerung der Mittelachse der Sägezahnreihe, welche in diesem Fall auch die Mittelachse des einen freien/distalen Schenkels (an welchem die Sägezahnreihe ausgebildet/angeordnet ist) darstellt, den Kupplungsbereich des Sägeblatts, vorzugsweise die Rotationsachse der Werkzeugaufnahme schneidet, sodass sich die Sägezahnreihe tangential längs der gewählten Kreisbahn hin- und herbewegt. An dieser Stelle sei noch darauf hingewiesen, dass unter dem Begriff "Sägerichtung" jene Richtung zu verstehen ist, die senkrecht zur Sägezahnreihe verläuft, also der Mittelachse der Sägezahnreihe bzw. des einen freien/distalen Schenkels der S-Form entspricht.

In anderen Worten ausgedrückt, hat das erfindungsgemäße Sägeblatt zum Durchtrennen von Knochen gemäß einem ersten Aspekt der vorliegenden Erfindungen einen Kupplungsbereich zum Einschieben (und Verrasten) in die Werkzeugaufnahme bzw. Einschubkupplung der Knochensägemaschine. Der Kopplungsbereich legt durch seine Ausgestaltung eine bestimmte Einschubrichtung fest, entlang der das Sägeblatt in die Werkzeugaufnahme der Knochsägemaschine eingeschoben werden kann. Beispielsweise hat die Werkzeugaufnahme hierfür eine Art Schwalbenschwanzführung oder zwei sich gegenüberliegende Klemmschienen/Klemmbacken, zwischen denen das Sägeblatt eingeschoben wird, um so Drehmomente auf das Sägeblatt übertragen zu können. Ferner kann zwischen den Klemmbacken eine Rastnase oder ein Rücksprung ausgebildet sein, vorzugsweise im Bereich (auf) der Rotationsachse der Werkzeugaufnahme, die mit einem entsprechend geformten Rastelement/Rastabschnitt am Sägeblatt im vollständig eingeschobenen Zustand verrastet und so ein unbeabsichtigtes Herausziehen des Sägeblatts aus der Werkzeugaufnahme verhindert.

Schließlich ist die (durch die Säge vorgegebene) Dreh-/Rotationsachse festgelegt, welche innerhalb des Kupplungsbereichs liegt, derart, dass bei einer oszillierenden Rotationsbewegung der Werkzeugaufnahme/Einschubkupplung um deren Rotationsachse das eingeschobene Sägeblatt eine oszillierende Drehbewegung zusammen mit der Werkzeugaufnahme/Einschubkupplung ausführt, bei der sich die Sägezahnreihe im Wesentlichen tangential entlang bzw. auf einer Kreisbahn längsbewegt. Die Sägezahnreihe (auch als Sägeverzahnung bezeichnet), die demzufolge zumindest im Wesentlichen entlang einer Tangentialrichtung bezogen auf die Dreh-/Rotationsachse der Werkzeugaufnahme/Einschubkupplung verläuft, ist so ausgerichtet, dass deren definierte Sägevorschubrichtung bzw. Zahnreihen-Mittelachse (im Wesentlichen senkrecht zur Sägeverzahnungsrichtung) in einem Winkel ungleich 180° (bzw. 0°) zu der Einschubrichtung, vorzugsweise in einem Bereich zwischen 15° und 80°, insbesondere 30° bis 60° und weiter vorzugsweise um 45° (+/- 5° Toleranz) verdreht ist.

Ein weiterer Kern der Erfindung, der ggf. unabhängig oder in Kombination mit den vorstehenden Merkmalen beansprucht werden soll, sieht vor, die Sägezahnreihe (Sägeverzahnung) nicht über die gesamte distale freie Stirnkante des erfindungsgemäßen Sägeblatts verlaufen zu lassen sondern zwischen zwei nicht gezahnten Endabschnitten der distalen freien Stirnkante einzufügen, wobei die beiden nicht gezahnten Endabschnitte der distalen freien Stirnkante unter Ausbildung jeweils einer nach vorn/seitlich konvexen Abrundung in die Seitenkanten des Sägeblatts übergehen. Dadurch wird ein Eingreifen der stirnseitigen Sägeverzahnung und insbesondere deren letzte Zähne in das Weichteilgewebe weitgehend auch dann verhindert, wenn die Maschine (unbeabsichtigt) geschwenkt wird.

Die Erfindung wird nachstehend anhand eines bevorzugten Ausführungsbeispiels unter Bezugnahme auf die begleitenden Figuren näher erläutert.

### Kurzbeschreibung der Figuren

Es zeigen:
Fig. 1 die Draufsicht einer Knochensägemaschine mit aufgestecktem Sägeblatt gemäß einem bevorzugten Ausführungsbeispiel der vorliegenden Erfindung,
Fig. 2 die Seitenansicht der Knochensägemaschine von Fig. 1 mit aufgestecktem Sägeblatt gemäß dem bevorzugten Ausführungsbeispiel der vorliegenden Erfindung,
Fig. 3 die rechtsseitige Draufsicht des Sägeblatts gemäß dem bevorzugten Ausführungsbeispiel der vorliegenden Erfindung,
Fig. 4 die linksseitige Draufsicht des Sägeblatts gemäß dem bevorzugten Ausführungsbeispiel der vorliegenden Erfindung und
Fig. 5 die Seitenansicht einer Knochensägemaschine mit aufgestecktem Sägeblatt gemäß dem Stand der Technik

### Figurenbeschreibung

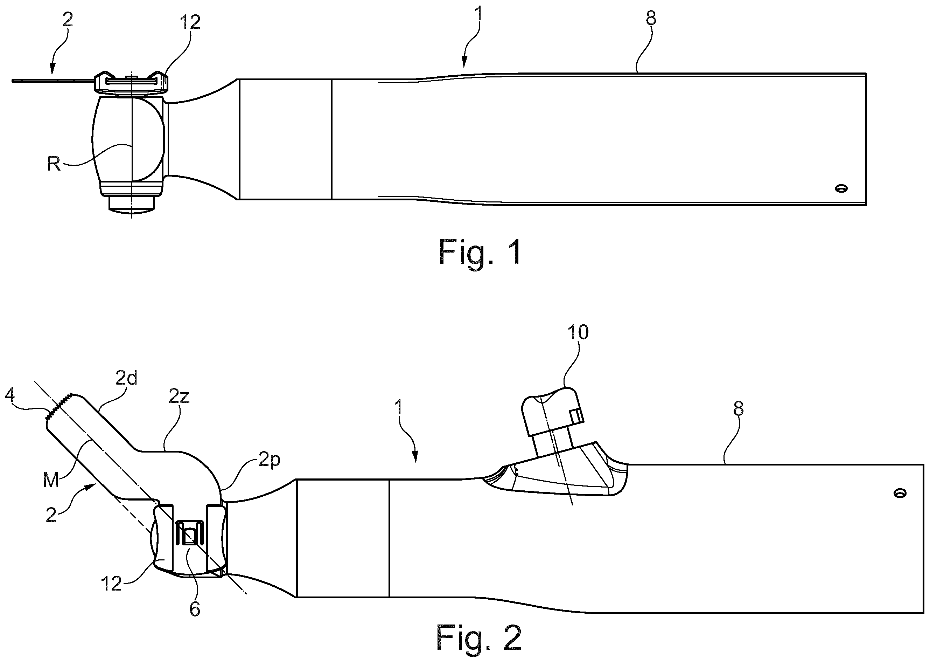

In den Fig. 1 und 2 ist eine Knochensägemaschine 1 gezeigt mit einem vorzugsweise auswechselbaren Sägeblatt 2 gemäß einem bevorzugten Ausführungsbeispiel der vorliegenden Erfindung, das im Gegensatz zu einem bekannten Sägeblatt nicht gerade sondern mit zwei (gegenläufigen) Abkrümmungen oder Biegungen etwa nach Art einer S-Form ausgebildet ist.

Das erfindungsgemäße Sägeblatt 2 erhält somit einen ersten distalen Schenkel 2d und einem zweiten proximalen Schenkel 2p, die in einem Winkel ungleich 0° bzw. ungleich 180° (vorliegend in etwa 45°) zueinander ausgerichtet sind. Die beiden Schenkel 2d, 2p des quasi S-förmig gewundenen Sägeblatts 2 sind über einen Zwischensteg 2z miteinander (stoffeinstückig) verbunden.

Der erste distale Schenkel 2d ist vorzugsweise gerade ausgebildet und hat eine distale Stirnkante, die mit einer Sägeverzahnung / Sägezahnreihe 4 von vorzugsweise kleiner/gleich 20mm Zahnreihenlänge (zwischen den jeweils äußersten Zähnen gemessen) ausgebildet oder versehen ist, die sich in etwa rechtwinklig zu einer Mittelachse M des ersten distalen Schenkels 2d erstreckt. Der zweite, proximale Schenkel 2p ist ebenfalls vorzugsweise gerade ausgebildet und hat in seinem distalen Endabschnitt einen Kopplungsbereich 6, wie er bereits aus dem Stand der Technik gemäß vorstehender Beschreibung bekannt ist. Daher kann an dieser Stelle auf die vorstehenden Beschreibungstextteile verwiesen werden. Dieser Kopplungsbereich 6 ist demzufolge dafür vorgesehen und ausgebildet mit der (allgemein bekannten) Werkzeugaufnahme/Einschubkupplung einer Knochensägemaschine in (Rast-)Eingriff zu kommen.

Eine derartige Knochensägemaschine 1 ist symbolisch in den Fig. 1 und 2 dargestellt und hat vorliegend ein hülsenförmiges Gehäuse 8, das auch als Griffstück dient und welches mit Betätigungshandhaben wie Druckschalter 10 oder dergleichen bestückt ist. Der darin aufgenommene Motor (nicht dargestellt) treibt, wie vorstehend anhand des Stands der Technik beschrieben, die Werkzeugaufnahme/Einschubkupplung 12 für deren oszillierende Rotationbewegung um eine Rotations-/Drehachse R an, die auf das fest mit der Werkzeugaufnahme 12 verrastete Sägeblatt 2 übertragen wird. Die Sägezahnreihe 4 führt dabei eine Hin- und Herbewegung längs einer Kreisbahn K aus, deren Mittelpunkt die Drehachse R der Werkzeugaufnahme bildet.

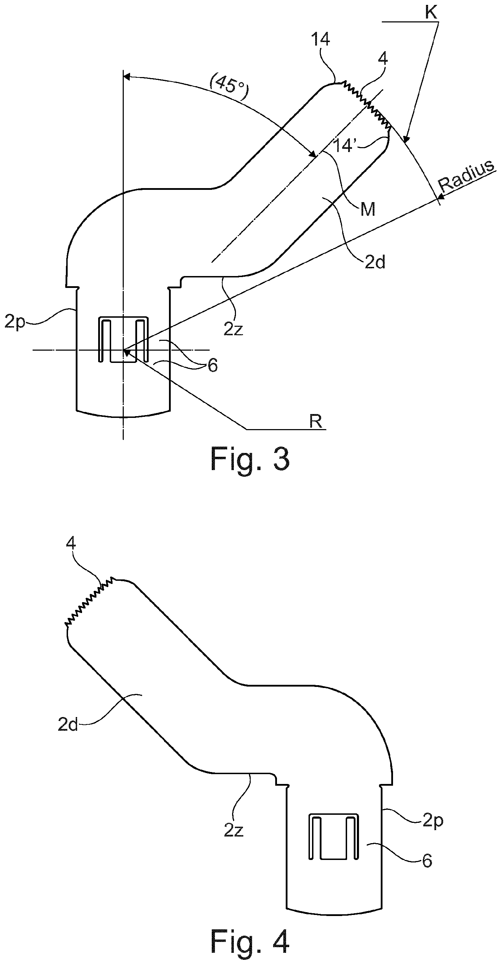

Damit die Sägezahnreihe 4 trotz der quasi S-Form des Sägeblatts 2 im Wesentlichen tangential zur Kreisbahn K ausgerichtet ist, ist der vorstehend beschriebene Zwischensteg 2z gemäß der Fig.3 und 4 so ausgebildet, dass er sich vorzugsweise rechtwinklig zu dem zweiten, proximalen Schenkel 2p erstreckt und eine Länge hat/erhält, die so gewählt ist, dass eine Mittelachse M des ersten distalen Schenkels 2d (in deren gedachter Verlängerung) die Rotationsachse R der Werkzeugaufnahme 12 bzw. Einschubkupplung schneidet, wenn das Sägeblatt in korrekter Weise in die Werkzeugaufnahme eingesetzt ist. Im vorliegenden Beispiel liegt der Drehpunkt des Sägeplatts in dessen Koppelbereich, vorzugsweise der Rastzunge, die im proximalen Schenkel 2p ausgebildet ist.

Die Sägezahnreihe 4 ist nicht geradlinig sondern gekrümmt, wobei deren Krümmungsradius im Wesentlichen jenem der Kreisbahn K entspricht, entlang derer sie sich hin- und herbewegt. Des Weiteren ist die Sägezahnreihe 4 gemäß der Fig. 3 und 4 kürzer als die Länge der distalen Stirnkante des Sägeblatts 1, sodass sich an den beiden Enden der Sägezahnreihe 4 jeweils ein unverzahnter Restabschnitt 14, 14' der distalen Stirnkante anschließt/verbleibt. Diese beiden Restabschnitte 14, 14'sind in Richtung distal und in Richtung seitlich konvex abgerundet und gehen so in einer Bogenform in die jeweiligen Seitenkanten des Sägeblatts 1 über. An dieser Stelle sei darauf hingewiesen, dass sämtliche Kantenübergänge zwischen den beiden Schenkeln und dem Zwischenstück ebenfalls abgerundet sind, um ein Verletzungsrisiko zu minimieren.

Abschließend soll noch erwähnt werden, dass die in den Fig. 1 und 2 dargestellte Knochensägemaschine nur beispielhaft ist und durch eine Knochensägemaschine gemäß der Fig. 5 ersetzt werden kann. Auch ist die dargestellte sowie eingangs beschriebene Kopplung zwischen Sägeblatt und Werkzeugaufnahme zwar in vielen Sägeschneidmaschinen vorzufinden (und damit allgemein bekannter gängiger Stand der Technik). Diese hat jedoch lediglich die Funktion, das Sägeblatt ggf. auswechselbar mit der Werkzeugaufnahme zu koppeln und kann durch eine andere Kopplungskonstruktion ersetzt werden, ohne dass hierdurch der Kern der vorliegenden Erfindung beeinflusst werden würde. D.h. unabhängig davon, wie die Kopplung des Sägeblatts mit der Werkzeugaufnahme erfolgt, ist es grundsätzlich möglich, durch die quasi S-Form des Sägeblatts den proximalen Schenkel individuell an die jeweilige Kopplung auszurichten und den distalen Schenkel beispielsweise auf der dargestellten Neigungsrichtung bezüglich der Werkzeugaufnahme bzw. der Maschinenlängsachse zu belassen, wobei die Länge des Zwischenstücks individuell so eingestellt werden kann, dass die Mittelachse des distalen Schenkels die Rotationsachse der Werkzeugaufnahme im Bereich der Kopplung schneidet.

Die vorliegende Erfindung betrifft zusammenfassend ein vorzugsweise auswechselbares Sägeblatt einer Knochensägemaschine mit einer distal angeordneten oder ausgebildeten Sägezahnreihe und einem proximal angeordneten oder ausgebildeten Kopplungsbereich zum Koppeln des Sägeblatts mit einer oszillierend rotierenden Werkzeugaufnahme der Knochensägemaschine, wobei das Sägeblatt mit zwei gegenläufigen Abkrümmungen oder Biegungen etwa nach Art einer S-Form ausgebildet ist.

## Patentansprüche

1. Sägeblatt für eine Knochensägemaschine (1) mit einem proximal angeordneten oder ausgebildeten sowie eine Kopplungs- oder Einschubrichtung definierenden Kopplungsbereich (6) zum Koppeln oder Einschieben des Sägeblatts (2) mit/in eine(r) oszillierend um eine Rotationsachse (R) rotierenden Werkzeugaufnahme (12) der Knochensägemaschine (1) und mit einer Sägezahnreihe (4), die an einer distalen Stirnkante des Sägeblatts (1) angeordnet oder ausgebildet ist, derart, dass deren senkrecht zur Sägezahnreihe (4) ausgerichtete Mittelachse (M) den Kopplungsbereich (6) und vorzugsweise die Rotationsachse (R) schneidet, wenn das Sägeblatt (2) mit der Werkzeugaufnahme (12) gekoppelt oder in diese eingeschoben ist, **dadurch gekennzeichnet, dass** die Mittelachse (M) und die Kupplungs- oder Einschubrichtung in einem Winkel ungleich 0° bzw. 180°, vorzugsweise in einem Bereich von 15° und 60°, weiter vorzugsweise in einem Bereich von 30° und 60° und weiter vorzugsweise in etwa 45° zueinander stehen und dass das Sägeblatt (2) mit zwei gegenläufigen Abkrümmungen oder Biegungen nach Art einer S-Form ausgebildet ist.

2. Sägeblatt nach Anspruch 1, **dadurch gekennzeichnet, dass** das Sägeblatt (2) durch die Ausbildung der beiden gegenläufigen Abkrümmungen oder Biegungen einen ersten distalen Schenkel (2d) und einen zweiten proximalen Schenkel (2p) aufweist, die in einem Winkel ungleich 0° bzw. ungleich 180° vorzugsweise in etwa 45° zueinander ausgerichtet sind.

3. Sägeblatt nach Anspruch 2, **dadurch gekennzeichnet, dass** die beiden Schenkel (2d, 2p) des Sägeblatts (2) über einen Zwischensteg (2z) miteinander vorzugsweise stoffeinstückig verbunden sind.

4. Sägeblatt nach einem der Ansprüche 2 und 3, **dadurch gekennzeichnet, dass** der erste distale Schenkel (2d) gerade ausgebildet ist und eine distale Stirnkante hat, die mit der Sägezahnreihe (4) ausgebildet oder versehen ist, wobei die Mittelachse (M) der Sägezahnreihe (4) gleichzeitig die Mittelachse des ersten distalen Schenkels (2d) ist und der zweite, proximale Schenkel (2p) ebenfalls gerade ausgebildet ist und in seinem proximalen Endabschnitt den Kopplungsbereich (6) aufweist oder bildet.

5. Sägeblatt nach Anspruch 4, **dadurch gekennzeichnet, dass** die Sägezahnreihe (4) im Wesentlichen tangential zu einer Kreisbahn (K) ausgerichtet ist, entlang derer sie sich bei einer oszillierenden Rotationsbewegung der Werkzeugaufnahme (12) in einem in die Werkzeugaufnahme (12) eingesetzten Zustand hin- und herbewegt, wofür der Zwischensteg (2z) eine solche Länge hat oder erhält, dass die Mittelachse (M) des ersten distalen Schenkels (2d) den Kopplungsbereich (6), vorzugsweise die Rotationsachse (R) der Werkzeugaufnahme (12) im Wesentlichen schneidet, wenn das Sägeblatt (2) mit der Werkzeugaufnahme (12) gekoppelt oder in diese eingeschoben ist.

6. Sägeblatt nach einem der vorstehenden Ansprüche, **dadurch gekennzeichnet, dass** die Sägezahnreihe (4) gekrümmt ist, wobei deren Krümmungsradius im Wesentlichen jenem der Kreisbahn (k) entspricht, entlang derer sie sich bei einer oszilierenden Rotation der Werkzeugaufnahme (12) in einem in die Werkzeugaufnahme (12) eingesetzten Zustand hin- und herbewegt.

7. Sägeblatt nach einem der vorstehenden Ansprüche, **dadurch gekennzeichnet, dass** das Sägeblatt ein medizinischen, vorzugsweise chirurgisches Werkzeug der Minimal-Invasiv-Bauart ist.

8. Sägeblatt nach Anspruch 7, **dadurch gekennzeichnet, dass** die Sägezahnreihe (4) eine Länge von kleiner/gleich 20 mm hat.

9. Sägeblatt nach einem der vorstehenden Ansprüche, **dadurch gekennzeichnet, dass** die Sägezahnreihe (4) kürzer ist als die Länge der distalen Stirnkante des Sägeblatts (2), an der die Sägezahnreihe (4) ausgebildet oder angeordnet ist, sodass sich an den beiden Enden der Sägezahnreihe (4) jeweils ein unverzahnter Restabschnitt (14, 14') der distalen Stirnkante anschließt oder verbleibt

10. Sägeblatt nach Anspruch9, **dadurch gekennzeichnet, dass** die beiden Restabschnitte (14, 14') in Richtung distal und in Richtung seitlich konvex abgerundet sind und so in einer Bogenform in die jeweiligen Seitenkanten des Sägeblatts (2) übergehen.

11. Chirurgische Knochensägevorrichtung mit einer Knochensägemaschine (1) die einen in einem Maschinengehäuse (8) aufgenommenen Antrieb, vorzugsweise Elektromotor hat, der mit einer Werkzeugaufnahme (12) zu deren oszillierenden Rotation wirkverbunden ist, die an einem distalen Ende des Maschinengehäuses (8) gelagert ist und einem Sägeblatt (2), das auswechselbar mit der Werkzeugaufnahme (12) gekoppelt oder in diese eingeschoben ist, derart, dass das Sägeblatt (2) zusammen mit der Werkzeugaufnahme (12) oszillierend rotiert, **dadurch gekennzeichnet, dass** das Sägeblatt (2) die Merkmale gemäß einem der vorstehenden Ansprüche aufweist.

## Claims

1. Saw blade for a bone saw machine (1) with a proximally arranged or formed coupling region (6) that defines a coupling or insertion direction, for coupling or inserting the saw blade (2) with/into an tool holder (12) of the bone saw machine (1), rotating in an oscillating manner about an axis of rotation (R) and with a row of saw teeth (4) that are arranged or formed at a distal end edge of the saw blade (1) such that the centre axis (M) thereof orientated perpendicular to the row of saw teeth (4) intersects the coupling region (6), and preferably the axis of rotation (R), if the saw blade (2) is coupled with the tool holder (12) or inserted therein, **characterised in that** the centre axis (M) and the coupling or insertion direction are at an angle that is not equal to 0° or 180°, preferably in a range between 15° and 60°, more preferably in a range between 30° and 60° and still more preferably at approximately 45° to one another, and **in that** the saw blade (2) is formed with two counter-rotating curvatures or bends in the manner of an S-shape.

2. Saw blade according to claim 1, **characterised in that** the saw blade (2), through the forming of the two counter-rotating curvatures or bends, has a first distal leg (2d) and a second proximal leg (2p), that are orientated at an angle that is not equal to 0° and not equal to 180°, preferably at approximately 45° to one another.

3. Saw blade according to claim 2, **characterised in that** the two legs (2d, 2p) of the saw blade (2) are connected together via an intermediate leg (2z) preferably as one piece.

4. Saw blade according to any of claims 2 and 3, **characterised in that** the first distal leg (2d) is formed straight and has a distal end edge, which is formed or provided with the row of saw teeth (4), wherein the centre axis (M) of the row of saw teeth (4) simultaneously forms the centre axis of the first distal leg (2d) and the second, proximal leg (2p) is likewise formed straight and comprises or forms the coupling region (6) in its proximal end section.

5. Saw blade according to claim 4, **characterised in that** the row of saw teeth (4) is orientated substantially tangential to a circular path (K), along which it reciprocates during an oscillating rotational movement of the tool holder (12) in an inserted state in the tool holder (12), for which purpose the intermediate leg (2z) has or obtains such a length that the centre axis (M) of the first distal leg (2d) substantially intersects the coupling region (6), preferably the axis of rotation (R) of the tool holder (12), if the saw blade (2) is coupled with the tool holder (12) or inserted therein.

6. Saw blade according to any of the preceding claims, **characterised in that** the row of saw teeth (4) is curved, wherein the radius of curvature thereof substantially corresponds to the circular path (k) along which it reciprocates during an oscillating rotation of the tool holder (12) in an inserted state in the tool holder (12).

7. Saw blade according to any of the preceding claims, **characterised in that** the saw blade is a minimally-invasive medical, preferably surgical, tool.

8. Saw blade according to claim 7, **characterised in that** the row of saw teeth (4) has a length less than or equal to 20 mm.

9. Saw blade according to any of the preceding claims, **characterised in that** the row of saw teeth (4) is shorter than the length of the distal end edge of the saw blade (2) on which the row of saw teeth (4) is formed or arranged, so that, in each case, an un-toothed residual section (14, 14') of the distal end edge adjoins or remains at the two ends of the row of saw teeth (4).

10. Saw blade according to claim 9, **characterised in that** the two residual sections (14, 14') are convexly rounded in the distal direction and in the lateral direction and thus pass into an arc shape in the respective side edges of the saw blade (2).

11. Surgical bone saw device with a bone saw machine (1) that has a drive, preferably an electric motor, accommodated in a machine housing (8), which is in working connection with a tool holder (12) for oscillating rotation thereof, which is mounted at a distal end of the machine housing (8), and a saw blade (2) that is exchangeably coupled with or inserted in the tool holder (12) such that the saw blade (2) is rotated in an oscillating manner together with the tool holder (12), **characterised in that** the saw blade (2) has the features according to any of the preceding claims.

## Revendications

1. Lame de scie pour une machine de type scie à os (1) avec une zone de couplage (6) agencée ou formée au niveau proximal et définissant une direction de couplage ou d'insertion pour le couplage ou l'insertion de la lame de scie (2) avec/dans un réceptacle d'outil (12) de la machine de type scie à os (1) tournant de façon oscillante autour d'un axe de rotation (R) et avec une rangée de dents de scie (4) qui est agencée ou formée au niveau d'un bord frontal distal de la lame de scie (1), de telle sorte que son axe médian (M) orienté perpendiculairement à la rangée de dents de scie (4) coupe la zone de couplage (6) et de préférence l'axe de rotation (R), lorsque la lame de scie (2) est couplée au réceptacle d'outil (12) ou est insérée dans celui-ci, **caractérisée en ce que** l'axe médian (M) et la direction de couplage ou d'insertion se situent dans un angle différent de 0° ou 180°, de préférence dans une plage de 15° et 60°, de manière davantage préférée dans une plage de 30° et 60° et de manière encore davantage préférée d'environ 45° l'un par rapport à l'autre et **en ce que** la lame de scie (2) est formée de deux courbures ou incurvations contraires à la manière d'un S.

2. Lame de scie selon la revendication 1, **caractérisée en ce que** la lame de scie (2) présente par la formation des deux courbures ou incurvations contraires, une première branche distale (2d) et une deuxième branche proximale (2p) qui sont orientées l'une par rapport à l'autre en un angle différent de 0° ou différent de 180°, de préférence d'environ 45°.

3. Lame de scie selon la revendication 2, **caractérisée en ce que** les deux branches (2d, 2p) de la lame de scie (2) sont liées l'une à l'autre par une traverse (2z) de préférence en un matériau d'un seul tenant.

4. Lame de scie selon l'une quelconque des revendications 2 et 3, **caractérisée en ce que** la première branche distale (2d) est formée de façon linéaire et a un bord frontal distal qui est formé ou doté de la rangée de dents de scie (4), dans laquelle l'axe médian (M) de la rangée de dents de scie (4) est en même temps l'axe médian de la première branche distale (2d) et la deuxième branche proximale (2p) est également formée de façon linéaire et présente ou forme la zone de couplage (6) dans sa section d'extrémité proximale.

5. Lame de scie selon la revendication 4, **caractérisée en ce que** la rangée de dents de scie (4) est orientée sensiblement de façon tangentielle par rapport à une trajectoire circulaire (K), le long de laquelle elle exerce un mouvement de va-et-vient lors d'un mouvement de rotation oscillant du réceptacle d'outil (12) dans un état introduit dans le réceptacle d'outil (12), à cette fin la traverse (2z) a ou adopte une longueur telle que l'axe médian (M) de la première branche distale (2d) coupe sensiblement la zone de couplage (6), de préférence l'axe de rotation (R) du réceptacle d'outil (12) lorsque la lame de scie (2) est couplée dans le réceptacle d'outil (12) ou insérée dans celui-ci.

6. Lame de scie selon l'une quelconque des revendications précédentes, **caractérisée en ce que** la rangée de dents de scie (4) est courbée, dans laquelle son rayon de courbure correspond sensiblement à celui de la trajectoire circulaire (k), le long de laquelle elle exerce un mouvement de va-et-vient lors d'une rotation oscillante du réceptacle d'outil (12) dans un état introduit dans le réceptacle d'outil (12).

7. Lame de scie selon l'une quelconque des revendications précédentes, **caractérisée en ce que** la lame de scie est un outil médical, de préférence chirurgical de type mini-invasif.

8. Lame de scie selon la revendication 7, **caractérisée en ce que** la rangée de dents de scie (4) a une longueur inférieure ou égale à 20 mm.

9. Lame de scie selon l'une quelconque des revendications précédentes, **caractérisée en ce que** la rangée de dents de scie (4) est plus courte que la longueur du bord frontal distal de la lame de scie (2) au niveau de laquelle la rangée de dents de scie (4) est formée ou agencée, de sorte qu'aux deux extrémités de la rangée de dents de scie (4), il reste ou il s'adjoint respectivement un segment résiduel non denté (14, 14') au bord frontal distal.

10. Lame de scie selon la revendication 9, **caractérisée en ce que** les deux segments résiduels (14, 14') sont arrondis de façon convexe en direction distale et en direction latérale et ainsi, se confondent en une forme d'arc dans les bords latéraux respectifs de la lame de scie (2).

11. Dispositif chirurgical de type scie à os avec une machine de type scie à os (1) qui a un entraînement, de préférence un moteur électrique, reçu dans un logement de machine (8), qui est lié fonctionnellement avec un réceptacle d'outil (12) pour sa rotation oscillante, qui est monté au niveau d'une extrémité distale du logement de machine (8) et une lame de scie (2) qui est couplée de façon interchangeable avec le réceptacle d'outil (12) ou insérée dans celui-ci de telle sorte que la lame de scie (2) tourne de façon oscillante conjointement au réceptacle d'outil (12), **caractérisé en ce que** la lame de scie (2) présente les caractéristiques selon l'une quelconque des revendications précédentes.
